# EUROPEAN PATENT APPLICATION

(11) **EP 0 916 367 A1**
(43) Date of publication of application: **19.05.1999**
(21) Application number: 98203401.9
(22) Date of filing: 12.10.1998
(51) Int. Cl.: A61N 1/40

(54) **Device for generating electro-magnetic oscillations for transmission to the body of a human or an animal**

(30) Priority: 05.11.1997 NL 1007451
(71) Applicant: Smarty B.V., 3852 NZ Speuld (NL)
(72) Inventor: van den Brink, Jan Willem, 3852 NZ Speuld (NL)
(74) Representative: Lips, Hendrik Jan George, Ir.

(57) **Abstract**

Device for generating electromagnetic oscillations for transmitting them to the body of a human or an animal, in order to exert a therapeutic effect. The generator (4) for generating the oscillations is situated in a container (1) that can possibly be carried along by a person. The container (1) contains a space (5) for receiving threads (6) of wool, cotton, silk or a like material and of a certain colour or combination of colours, or for receiving another substance or combination of substances suitable for the purpose, for influencing the oscillations generated by the generator (4) before they are supplied to the body. The container can be provided with signalling and control means (18, 19). The oscillations are transmitted by a cable (12) from the container (1) to a resilient wrist, arm or ankle strap (15) that can have a conductive part contacting the skin. With the wireless version, these can be contact points.

## Description

The invention firstly relates to a device for generating electromagnetic oscillations for transmitting them to the body of a human or an animal, for therapeutic purposes, said device comprising a container containing at least the generator for generating the oscillations, in which the generated oscillations can be transmitted to the body directly or indirectly from said generator.

Such a device is known from US 5 401 233. By such a device, electromagnetic oscillations can be transmitted to the human body, but possibly the animal body too, in order to realize a therapeutical effect. With the known device, this concerns body parts such as the back of the neck, for example, to which the oscillations are transmitted.

Until recently, it was assumed that bacteria could be fought effectively with the help of antibiotics, corticosteroids and like medicines. However, in practice more and more types of bacteria appear to have become therapy-resistent. The same goes for other pathogens. In connection with that, other treatments for very different complaints were looked for. Then, in some cases, treatments with electromagnetic oscillations produced satisfactory results.

It was found that not only the frequency of the oscillations, but the wavelength and the waveform and generally all characteristics of the oscillations are of great importance for obtaining the desired effect. Thus, for treating one complaint, the oscillations will have to have completely different characteristics than for treating another complaint.

The invention provides a device of the type described above, in which the container, in which the generator for generating the oscillations is situated, contains at least one space for accommodating threads of wool, silk or a like material and of a certain colour or combination of colours, or for accommodating another material or combination of materials suitable for the intended purpose, for influencing the oscillations generated by the generator before they are supplied to the body.

Further, the container can be provided with a power source and with signalling means such as for signalling that the energy of the power source has sunk beyond a certain value. It can also be signalled that the device is operative, e.g. by a light, flashing or otherwise, such as an LED. If a connection for mains or another energy source is provided for saving and possibly recharging the battery, the use of said connection can be signalled as well. Another energy source can be the battery of a car to which the device can be connected through the cigarette lighter. The latter can be done e.g. through an adapter, used at the same time for recharging batteries.

According to a further development, the container can be provided with means for continuously variable adjustment of the power supplied by the device, the number of pulses supplied by the device, and the like.

As it happens, not all cases require that the oscillations are supplied to the body in a continuous way. It is also possible to supply oscillations in a pulsating way so that the supply of oscillations to the body is always periodically interrupted. The time in which the oscillations are transmitted consecutively and the time in which the transmission is interrupted can possibly be set independently of each other. It is also possible to use a continous basic oscillation and superposing it, periodically, with a pulsating oscillation. Owing to this, a very definite waveform characteristic can be achieved. Then, in some cases, the period of treatment can be shortened and a more adequate operation of the device can be achieved. One can also provide for means for producing a signal indicating that a certain period of treatment or resonance process has elapsed.

In a wireless way or by a cable, the generated oscillations can be transmitted to means which are fixed on the skin of the body such as with the help of a sticking plaster.

In particular, the oscillations will be transmitted from the container by means of a cable to a resilient wrist, arm or ankle strap having at least the part that will contact the skin of the wrist, arm or ankle executed in an electrically conductive material.

Due to that, in many cases, the patient is able to control the use of the device himself/herself, if desired, without having to call someone to do it for him/her.

In particular, the cable will be provided with a contact plug at both ends and both the container and the wrist, arm or ankle strap will be provided with a contact socket in which the contact can be received. Naturally, the contact socket and plug can change positions.

Then, the cable can be led e.g. through a sleeve of a coat from a breast pocket to the wrist and then be connected to the container and the wrist strap. This facilitates the use of the device considerably.

It is also possible that by providing a number of spaces in which various threads can be inserted, various treatments can be performed at the same time or in succesion. In the latter case, only one cable is required, in which its contact plug can be plugged into one of the contact sockets of the container. The container will then be provided with more than one connecting plug or contact point. Then, it will be possible to treat a number of patients simulataneously. Obviously, it is also possible that one patient uses several separate devices at the same time for treating various complaints.

It has turned out, that by choosing threads of a specific colour, or threads of some specific colours and of a specific composition, or by choosing the appropriate substances for inserting into the container, such oscillations can be generated that resonance processes are generated at certain locations in the body. The resonance process acts on certain germs or the like which are brought in resonance and can thereby be deactivated. This can even be achieved with germs having settled in the core of a cell. The device operates like a filter and selects the proper characteristics of the oscillations being generated by the substances in the container or containers and supplies them to the body, where a resonance process is started.

One establishes by experiment, which colour or colours the threads or which properties the employed substances should have in a specific case. When this has been done for a specific case, it turns out that this colour or these colours or the other substances can also be employed with other patients having the same disease. For less frequently occurring ailments and with cases in which no adequate diagnosis can be made, it will of course require some time to be able to establish the proper threads or substances to be used.

Although the device according to the invention is firstly intended for the human body, the animal body can also be treated with it. Further, treatment of plants can also be contemplated, since they may also contain certain pathogens or the like that can be fought with electromagnetic oscillations.

Whereas generally high-frequency oscillations are employed, it is also possible to employ combinations of high and low frequencies. Due to this, oscillations having different characteristics can be generated.

The invention also relates to a method of treating the human or animal body using electromagnetic oscillations being transmitted to the body, in which the characteristics of said oscillations are influenced by employing threads of various materials, of various colours and of other substances including medicines, food supplements and all other substances that may have a beneficial influence on body and mind.

The invention is further explained by way of an example shown in the drawing, in which:

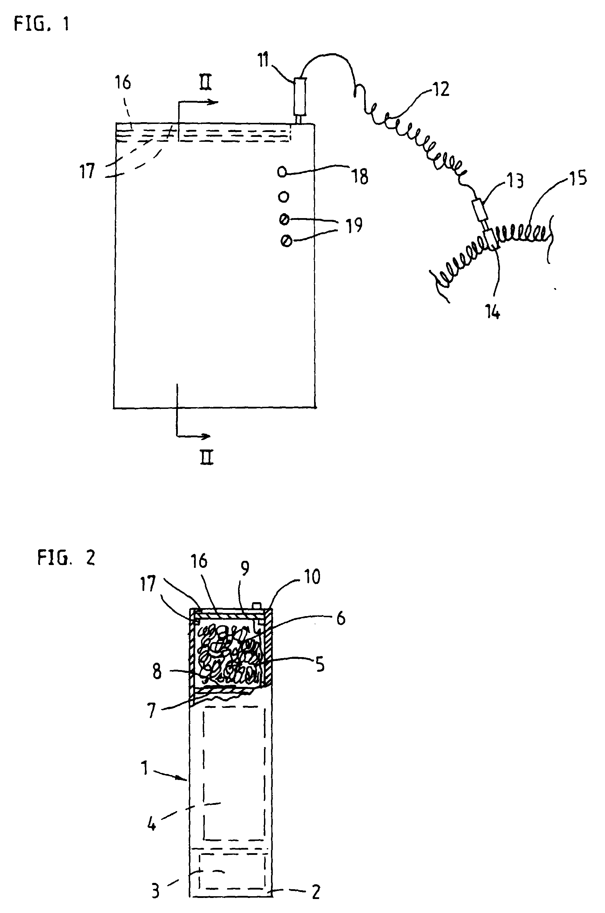

Fig. 1 shows a view of a device according to the invention and the cable connected to it, which in turn is connected to a strap for connecting to the body, only partly illustrated; and
Fig. 2 shows a view and a partial cross-section according to the line II - II of Fig. 1, in which the connecting strap has been left out.

The drawing shows a container 1 being provided with a chamber 2 for receiving a battery 3 or another energy source. Means for saving or recharging the battery can be present. The battery 3 is connected to a generator 4 for generating oscillations.

The container 1 further contains a space 5 in which threads 6 of wool, cotton, silk or a like material and of a certain colour or combination of colours can be inserted, or other substances including medicines, food supplements and all other substances capable of having a beneficial influences on body and mind.

Naturally, completely other kinds of materials can be employed, provided that they are capable of providing the desired effect. The threads 6 are in contact with a contact member 8 mounted on the inner wall 7 of said container and with a contact member 9. To that end, the wall 7 can consist of metal or of a material, such as plastic, onto which a conductive circuit has been mounted.

The container 1 is further provided with the contact socket 10 in which the contact plug 11 being connected to a cable 12 can be plugged. The other end of the cable 12 is provided with the contact plug 13 which can be plugged into the contact socket 14 being connected to a flexible metal wrist or arm strap 15. The strap 15 can be formed in a known way by approximately helically winding a metal wire or a small strip, in such a way that it has a flattened shape in cross-section. Naturally, the wrist strap can also be made of a flexible, non-conductive material and be provided with a part of conducting material which will contact the skin of the wearer. The strap can also be provided with a certain coating, such as of surgical steel, for example.

The space 5 of the container 1 can be closed off by means of a slider 16 incorporated between ribs 17, mounted on the lateral walls of said container.

The container can further be provided with a number of signalling means being indicated by 18 and being able to generate lighting or audio signals. They can serve for signalling that the power of the energy source has sunk below a certain level, for example.

Further, the container can be provided with means 19 for controlling the power supplied by the device, the number of pulses supplied by the device and the like.

The container can yet be provided with a clip or like member in order to be able to fix it, e.g. to a breast pocket, so that the person will not be limited to a fixed place for treatment.

It will be obvious, that only one single possible embodiment of a device according to the invention has been illustrated in the drawing and described above and that many changes can be made without leaving the inventive idea as it is defined in the claims.

## Claims

1. Device for generating electromagnetic oscillations for transmitting them to the body of a human or an animal, for therapeutic purposes, said device comprising a container (1) containing at least the generator (4) for generating the oscillations, in which the generated oscillations can be transmitted to the body directly or indirectly from said generator (4), characterized in that the container (1), in which the generator (4) for generating the oscillations is situated, contains at least one space (5) for accommodating threads (6) of wool, silk or a like material and of a certain colour or combination of colours, or for accommodating another material or combination of materials suitable for the intended purpose, for influencing the oscillations generated by the generator before they are supplied to the body.

2. Device according to claim 1, characterized in that the container (1) is provided with a power source and with signalling means (18) such as for signalling that the capacity of the power source (3) has sunk beyond a certain value and for like purposes.

3. Device according to claim 1 or 2, characterized in that the container (1) is provided with means (10) such as for continuously variable adjustment of the power supplied by the device, the number of pulses supplied by the device, and the like.

4. Device according to one of the preceding claims, characterized in that the oscillations will be transmitted, such as by means of a cable (12), from the container (1) to a resilient wrist, arm or ankle strap (15) having at least the part that will contact the skin of the wrist, arm or ankle executed in an electrically conductive material.

5. Device according to claim 4, characterized in that the cable (12) is provided with a contact plug (11, 13) at both ends and in that both the container (1) and the wrist, arm or ankle strap (15) are provided with a contact socket (10, 14) in which the contact plug (11, 13) can be received.

6. Method of treating the human or animal body using electromagnetic oscillations being transmitted to the body, characterized in that the characteristics of the oscillati-ons are influenced by employing threads (6) of various materials, of various colours and of other substances, including medicines, food supplements and all other substances capable of having a beneficial influence on body and mind.
